## Europäisches Patentamt

## European Patent Office

## Office européen des brevets

(19)

(11) Veröffentlichungsnummer: **0 221 199**
**B1**

(12) **EUROPÄISCHE PATENTSCHRIFT**

(45) Veröffentlichungstag der Patentschrift:
20.09.89

(51) Int. Cl.⁴: **A61N 5/06, A61H 33/04**

(21) Anmeldenummer: 85114154.9

(22) Anmeldetag: 07.11.85

(54) Vorrichtung zur balneo-phototherapeutischen Hautbehandlung.

(43) Veröffentlichungstag der Anmeldung:
13.05.87 Patentblatt 87/20

(45) Bekanntmachung des Hinweises auf die Patenterteilung:
20.09.89 Patentblatt 89/38

(84) Benannte Vertragsstaaten:
AT BE CH DE FR IT LI LU NL

(56) Entgegenhaltungen:
DE-A- 2 910 468
DE-A- 2 911 758
DE-A- 3 221 647
DE-A- 3 317 140
DE-U- 8 509 147

DE-Z.Fortschr. Med. 101.Jg. (1983), Nr. 20,
S. 931 bis 936 (Aufsatz von Dr. med. M. Ständer "Die
Thermalsole-Phototherapie.....");
DE-Z.Der Hautarzt 29, 328-330 (1978) (Aufsatz von Dr.
med. M.Ständer "Erfahrungen mit der
Thermalsole-Phototherapie.....");
DE-Z.Ärztliche Praxis, 34. Jg., Nr. 48, 15.6.1982,
Sonderdruck S. 3 bis 6 (Aufsatz von Dr. med. J. Arndt
"Salz aus dem Gelobten Land.....");
IT-Z. Pharmacological Research Communications,
Vol. 17, Nr. 6 (Juni) 1985, S. 501 und 503

(73) Patentinhaber: Schneider, Karl, Ostlandstrasse 12,
D-6427 Bad Salzschlirf(DE)

(72) Erfinder: Schneider, Karl, Ostlandstrasse 12, D-6427 Bad
Salzschlirf(DE)

(74) Vertreter: Linser, Heinz et al, Patentanwälte Heinz
Linser Dipl. Ing. Eckhardt Eyer
Robert-Bosch-Strasse 12a Postfach 10 22 10,
D-6072 Dreieich(DE)

## Beschreibung

Die Erfindung betrifft eine Vorrichtung zur balneo-phototherapeutischen Behandlung von Haut- oder rheumatischem Erkrankungen und/oder kosmetischen oder prophylaktischen Hautbehandlung.

Zur Behandlung der Psoriasis und ähnlicher Hautkrankheiten ist es bekannt, einen Ausschnitt des UV-Spektrums im Grenzbereich von UVB und UVA zu verwenden, der Wellenlängen von 290 bis 300 nm aufweist. Eine Vorrichtung dieser Art ist beispielsweise aus der DE-A 2 910 468 zu entnehmen.

Nach früherer Auffassung hat eine Strahlung, die länger als 330 nm ist, keine antipsoriatische Wirksamkeit mehr. Dies ist im Grundsatz zwar richtig, jedoch bringt die längerwellige Strahlung positive, ergänzende Wirkungen, auf die später näher eingegangen wird.

Dementsprechend sind auch Geräte bekannt, welche eine UV-Strahlungsvorrichtung mit einer Metall-Hochdrucklampe aufweisen, deren Füllung so gewählt ist, daß sich mehrere stark ausgeprägte Spektrallinien im Bereich zwischen 300 und 330 nm finden. Diese Lampe ist eine punktförmige Strahlungsquelle, mit der sich größere Flächen erst aus ausreichend großer Entfernung bestrahlen lassen.

Begingt durch eine intensive Entwicklung auf dem Gebiet der Heliotherapie sind heute eine Vielzahl von Reaktionen des Organismus auf die Applikation von optischer Strahlung bekannt, die zum Teil über die Beeinflussung des vegetativen Nervensystems und zum Teil durch photochemische Reaktionen in der Haut zustande kommen.

Entscheidend für alle diese Reaktionsabläufe sind die spektrale Verteilung der Strahlungsenergie und die Strahlendosis.

Während die langwellige Infrarot-Strahlung als ein wirksames Heilmittel bei Furunkulose, Rheumatismus und Erkältungen erkannt wurde, zeigt die sichtbare Strahlung über das Auge wirkend eine positive Beeinflussung der Psyche.

Besondere Bedeutung hat die therapeutische Anwendung von UV-Strahlung bei vielen Hautkrankheiten erreicht.

Hierbei ist der Wellenlängenbereich der optischen Strahlung von besonderer Bedeutung, der bei Absorption im Molekül Änderungen in der Elektronenschale bewirkt, ohne jedoch Ionisationsvorgänge einzuleiten. Positive Wirkungen können durch Strahlung im Wellenlängenbereich zwischen 300 bis 400 nm ausgelöst werden.

Kurzwelligere Strahlung als 290 nm sollte aus gesundheitlichen Gründen nicht eingesetzt werden und eine Strahlung, die langwelliger als 340 nm ist, zeigt allein eingesetzt keine Wirkung. In Kombination mit der Strahlung bis 300 nm liegen jedoch positive Wirkungen vor.

In den letzten Jahren wurden viele Bräunungsstudios eingerichtet, welche mit Sonnenbänken ausgerüstet sind, die zusätzlich mit einem auf- und abbewegbaren Sonnenhimmel ausgerüstet sein können, so daß eine Ganzkörperbestrahlung durchführbar ist. Eine übermäßige Bestrahlung kann Schäden hervorrufen, welche bis zur Hautkrebserkrankung führen können.

In der Zeitschrift "Der Hausarzt" 29, S. 328–330 (1979) wird über Erfahrungen mit der Thermalsole-Phototherapie bei der Psoriasis vulgaris berichtet. Hierbei kommt man zu dem Ergebnis, daß die Benetzung der Haut mit Flüssigkeit vor der Bestrahlung von besonderer Bedeutung sei, weil sich auf der Haut ausbildende Wassertropfen eine Brennglaswirkung aufweisen, welche eine Intensivierung der Strahlenwirkung hervorrufen.

Seit Menschengedenken ist die für den Körper positive Wirkung eines Bades bereits bekannt, insbesondere wenn das Bad mit Badesalzen angereichert und temperiert ist. Eine bevorzugte Stellung nimmt hier das Tote Meer ein. Seine Heilwirkung ist bereits über tausende von Jahren bekannt, und bedingt durch seine große Anreicherung an Salzen der unterschiedlichsten Art, erstreckt sich die Heilwirkung auf die verssschiedensten Krankheiten. Erst in jüngster Zeit wurden die in diesem Zusammenhang vorliegenden Probleme näher erforscht.

Es stellte sich heraus, daß das Tote Meer eine andere Salzzusammensetzung aufweist als die übrigen Meere, wie etwa das Mittelmeer, die Nordsee oder bekannte Sole-Quellen. So ist insbesondere der Anteil an Magnesium, Kalium und Brom weitaus höher. Dies allein ist jedoch für die Erklärung der Heilwirkung noch nicht ausreichend.

Es wurde festgestellt, das das Sonnenlicht im Bereich des Toten Meeres im Vergleich zur Globalstrahlung, d.h. die Strahlung, die direkt von der Sonne und gestreut von der Erdatmosphäre eine horizontale, ebene Erdoberfläche erreicht, erheblich abweicht. Es zeigt sich, daß die vergleichbaren Werte im Bereich von 300 bis 390 nm niedriger liegen als die Werte der Globalstrahlung. Bedingt durch die Lage des Toten Meeres ca. 400 m unter dem Meeresspiegel der übrigen Weltmeere ist für die Strahlung eine 400 m starke Luftschicht vorhanden, welche eine entsprechende Filterwirkung aufweist, wie sie an keiner anderen Stelle der Welt vorliegt. Im gleichen Verhältnis sind die Schwellenzeiten zum Erreichen der minimalen erythemwirksamen Dosis am Toten Meer größer als bei der Globalstrahlung.

Der Erfindung liegt die Erkenntnis zugrunde, daß die Kombination der Wirkung des im Bereich des Toten Meeres herrschenden Lichtspektrums der Sonne mit der spezifischen Zusammensetzung des Meerwassers eine besondere Heilkraft für die verschiedensten Krankheiten, insbesondere Hautkrankheiten, entwickelt, wie sich durch wissenschaftliche Untersuchungen bestätigen läßt.

Der Erfindung liegt eine weitere Erkenntnis zugrunde, nämlich daß die negative Wirkung der in den Sonnenstudios verwendeten Sonnenbänke und Sonnenhimmel dadurch vermieden werden kann, daß die Haut während der Bestrahlung periodisch mit einer Flüssigkeit benetzt wird, welche in Wasser gelöste Salze und damit Ionen, d.h. elektrisch geladenen Teilchen aufweist. Die durch die Strahlung aktivierte Haut wird für die Ionen sensibler und absorbiert diese.

Aus der DE-U 8 509 147 sind ferner bereits Wannen zur therapeutischen Behandlung mit Thermosole bekannt, welche Pumpvorrichtungen zur Umwälzung der Sole und isolierte Innenflächen aufweisen. Wannen dieser Art dienen jedoch nur für Vollbäder, so daß eine periodische Benetzung aller Körperpartien eines Patienten nicht möglich ist.

Der Erfindung liegt die Aufgabe zugrunde, eine Vorrichtung anzugeben, mit deren Hilfe ein Patient einer Strahlung unterworfen wird, welche der Sonnenstrahlung nahe kommt, die am Toten Meer vorherrscht, in Kombination mit einem Bad, welches eine Zusammensetzung aufweist, die der Zusammensetzung des Wasser des Toten Meeres entspricht, wobei das Austrocknen der Haut durch Strahlenwirkung verhindert oder vermindert werden soll.

Die Lösung dieser Aufgabe besteht gemäß der Erfindung darin, daß die eingangs genannte Vorrichtung mindestens einen Strahler mit einem Spektrum, welches den Spektralbereich von 290–420 nm umfaßt und mit einer Solebadvorrichtung deren Sole Spurenelemente sowie einen Salzgehalt von 5–20% und eine Temperatur von 20–38°C aufweist, und die eine Wanne aus einem säurebeständigen Material umfaßt, in der oberhalb des Wannenbodens eine fest eingebaute oder herausnehmbare Liegefläche angeordnet ist, so daß ein Becken mit einer Wassertiefe von 10–50 cm gebildet wird, wobei der oder die Strahler derart angeordnet sind, daß die Liegefläche der Solebadvorrichtung gleichmäßig ausgeleuchtet ist.

Mit dieser Vorrichtung nach der Erfindung wird in einer wechselnden Folge die Haut des Patienten mit einer günstigen Strahlung relativ kurzzeitig bestrahlt und unmittelbar anschließend in eine Flüssigkeit getaucht oder durch diese benetzt.

Die durch die Strahlung aktivierte Haut nimmt in der sich unmittelbar anschließenden Bade- oder Befeuchtungsphase die in Ionenform vorliegenden Salze bevorzugt auf und absorbiert diese, mit der Wirkung, daß durch das Eindringen dieser Mineralien, besonders der Magnesiumionen und Calciumionen, die Bildung des körpereigenen zyklischen Hormons (AMP) Adenylsäure in vorteilhafter Weise angeregt wird.

Die Zusammensetzung des Solebades weist gemäß der Erfindung bei einer Salinität von 299,89 die folgende Ionenkonzentration in mg pro Liter Wasser, mit einem Spielraum von +/–20% auf:

| | |
|---|---|
| Magnesium | 36,15 |
| Calcium | 13,78 |
| Natrium | 38,51 |
| Kalium | 7,26 |
| Strontium | 0,24 |
| Lithium | 0,01 |
| Chlorid | 194,44 |
| Bromid | 4,56 |
| Sulfat | 0,72 |
| Hydrogencarbonat | 0,23 |
| Sonstige Salze | 1,12 |

Das Solebad weist bei einem spezifischen Gewicht von 1,2 bis 1,3 in abgewandelter Darstellung die folgende Zusammensetzung in g pro Liter Wasser, mit einem Spielraum von +/–20% auf:

| | |
|---|---|
| Magnesiumchlorid | 280 |
| Calciumchlorid | 80 |
| Natriumchlorid | 25 |
| Brom | 8,3 |
| Calciumsulphat | 0,1 |
| Kaliumchlorid | 23 |

Im Kopfbereich der Wanne befinden sich ein Flüssigkeitseinlauf und im Fußbereich der Wanne ein Flüssigkeitsauslauf zur Umwälzung der Sole. Bei festem Einbau der Liegefläche ist zwischen Wannenboden und Liegefläche eine Pumpe mit angeschlossener thermostatisch geregelter Heizung angeordnet.

Damit läßt sich die Sole in der Wanne umpumpen, ohne daß hierzu eine zentrale Einrichtung erforderlich ist.

Durch diese Maßnahme ist eine individuelle Behandlung sowohl in einem Institut, einer Klinik als auch im Privatbereich möglich.

Der Flüssigkeitsauslauf der Solebadvorrichtung ist über eine Filtervorrichtung, einen Aufbereitungstank, einen thermisch isolierten Speichertank, eine thermostatisch gesteuerte Heizung und eine Pumpvorrichtung mit dem Flüssigkeitseinlauf der Solebadvorrichtung verbunden.

In Weiterbildung der Erfindung ist der Aufbereitungstank einer zentralen Anlage mit einem Frischwasserzulauf und einem Abwasserablauf über steuerbare Ventile verbunden. Hierdurch läßt sich das Solebad in der gewünschten Konzentration herstellen und nach Gebrauch aufbereiten. Die Sole wird ständig auf den Solegehalt in an sich bekannter Weise untersucht, und der Solegehalt wird danach entsprechend festgelegt.

Nach der Erfindung läßt sich eine wirtschaftlich arbeitende Anlage erstellen, welche für Kliniken oder Badehäuser einsetzbar ist. Hierfür ist es vorteilhaft, eine größere Anzahl Wannen zur Aufbereitungs- und Speichervorrichtung parallel zu schalten. Dadurch wird erreicht, daß alle Vorrichtungen zur Zubereitung der Sole und Aufbereitung einschließlich Filterung des gebrauchten Wassers zentral angeordnet und eingesetzt werden können, so daß eine wirtschaftlich arbeitende Anlage erstellt werden kann.

Für den privaten Einsatz der Erfindung lassen sich bestimmte Aggregate, wie oben ausgeführt, mit der Wanne integrieren, wie zum Beispiel Pumpen und Filter, welche unterhalb oder unmittelbar neben der Wanne eingebaut werden können.

Um das Verfahren nach der Erfindung durchführen zu können, ist über der Wanne mindestens ein mobiler oder stationärer Strahler angeordnet, der höhenverstellbar ausgebildet ist.

Die Erfindung wird anhand der Zeichnungen näher erläutert. Hierbei zeigen:

Figur 1 einen Querschnitt eines Beckens für ein Solebad mit einem integrierten Bewegungsbecken und einer Anordnung der Strahler über den ovalförmig um das Bewegungsbecken ausgebildeten Liegeflächen, in schematischer Darstellung;

Figur 2 eine Draufsicht auf ein Becken für ein Solebad mit einem integrierten Bewegungsbecken und einer Anordnung der Strahler über den Liegeflächen nach Figur 1, in schematischer Darstellung;

Figur 3 einen Querschnitt eines Beckens für ein Solebad mit einem integrierten Bewegungsbecken und einer Anordnung der Strahler über den parallel zum Bewegungsbecken ausgebildeten Liegeflächen, in schematischer Darstellung;

Figur 4 eine Draufsicht auf ein Becken für ein Solebad mit einem integrierten Bewegungsbecken und einer Anordnung der Strahler über der Liegeflächen nach Figur 3, in schematischer Darstellung;

Figur 5 einen Querschnitt durch eine als Becken ausgebildete Liegefläche;

Figur 6 eine Draufsicht auf eine als Becken ausgebildete Liegefläche nach Figur 5;

Figur 7 einen Querschnitt durch eine Wanne nach der Erfindung, und

Figur 8 eine schematische Darstellung einer zentralen Einrichtung mit den erforderlichen Einrichtungen zur Filterung, Aufbereitung, Speicherung und zum Transport;

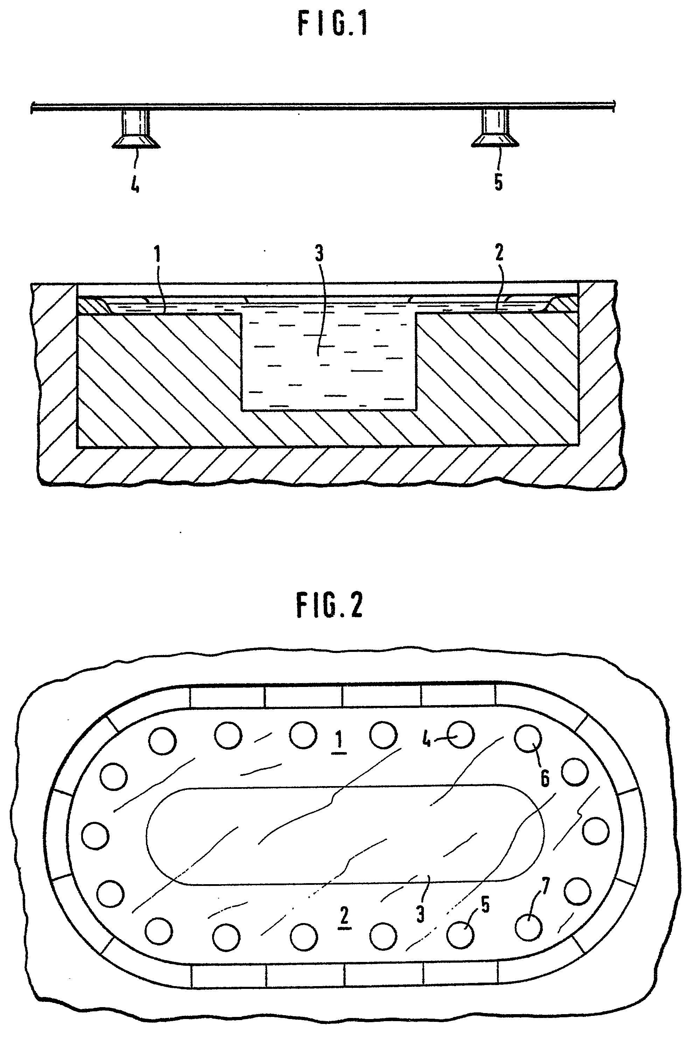

Die Figur 1 zeigt im Querschnitt eine Solebadvorrichtung, bestehend aus einem flachen Becken 1 mit einer Tiefe von 10–50 cm, und einem Bewegungsbecken 3, welches mit dem flachen Becken direkt verbunden ist. Auf der gegenüberliegenden Seite zum Becken 1 befindet sich ein weiteres flaches Becken 2 als Liegefläche. Oberhalb der Solebadvorrichtung sind mehrere Metallhalogenid-Hochdruckstrahler (MeH) 4, 5, 6 und 7 angeordnet, welche auch als Quecksilber-Hochdruck- (HgH) und/oder Quecksilber-Niederdruckleuchten (HgN) ausgebildet sein können.

Die genannten Strahler sind mit nicht näher dargestellten Filtereinrichtungen ausgerüstet, welche den Spektralbereich von 290 bis 400 nm durchlassen. Die Anordnung der Strahler ist derartig gewählt, daß die Liegeflächen 1 und 2 des Solebades ausgeleuchtet werden.

Die Tiefe des Bewegungsbeckens 3 beträgt etwa 1,20 bis maximal 1,50 m, so daß eine Person in dem Becken aufrecht stehen kann. Die Sole mit einem spezifischen Gewicht von 1,2 bis 1,3 hat einen entsprechend günstigen Auftrieb, so daß die Gelenke einer Person während der Bewegungstherapie vom eigenen Körpergewicht vollständig entlastet sind. Der Patient kann sich in bestimmten Zeitintervallen der Strahlung durch die Strahler 4 bis 7 direkt aussetzen, indem er sich in das flache Becken begibt. Die Tiefe dieses Beckens ist so gewählt, daß im liegenden Zustand die oberen Partien des Körpers aus dem Bad herausragen, so daß eine direkte Bestrahlung möglich ist. Durch regelmäßiges benetzen der Haut oder durch umdrehen des Körpers wird erreicht, daß die Haut während der Bestrahlung periodisch mit einer Flüssigkeit befeuchtet wird, welche die im Wasser gelösten und damit in Ionenform vorliegenden Salze aufweist. Die durch die Strahlung aktivierte Haut nimmt daher die in Ionenform vorliegenden Salze bevorzugt auf und absorbiert diese, wie eingangs bereits ausgeführt wurde, ohne daß die Haut austrocknet.

Die Figur 2 zeigt eine Draufsicht nach Figur 1, aus der ersichtlich ist, daß die Liegeflächen 1 und 2 eine geschlossene Fläche bilden und das Bewegungsbecken 3 umgeben. Die balneo-phototherapeutische Behandlung läßt sich mit dieser Ausführungsform mit einer Bewegungstherapie kombinieren.

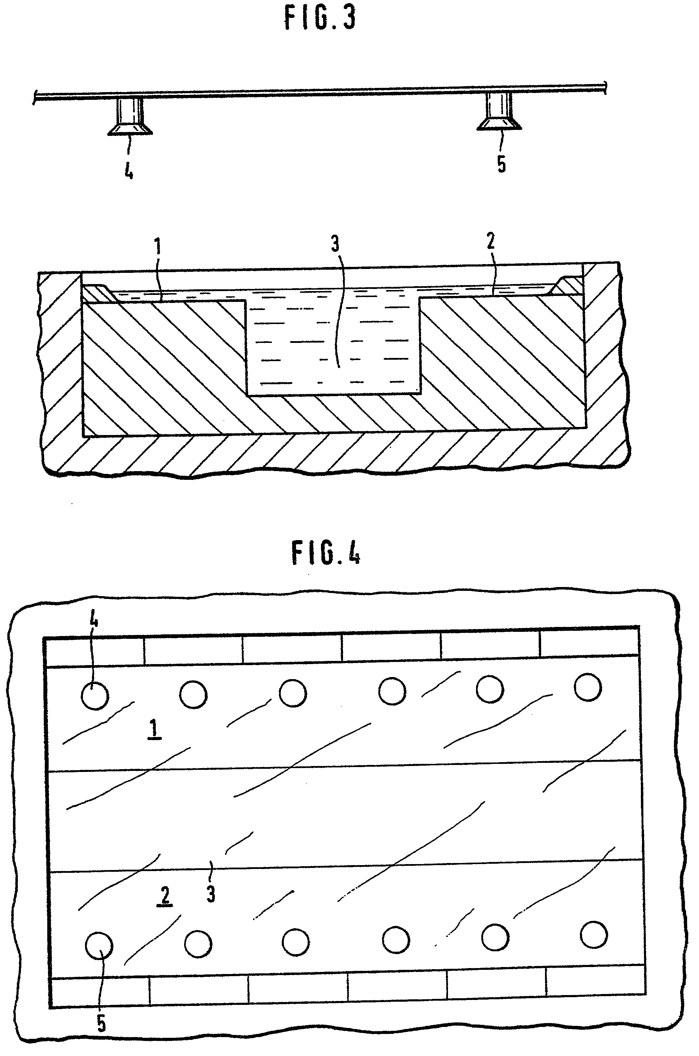

Die Figuren 3 und 4 zeigen abgewandelte Ausführungsformen der goemetrischen Ausgestaltung der Liegeflächen in Beziehung zum Bewegungsbecken. Die Liegeflächen 1 und 2 liegen hier zueinander und zum Bewegungsbecken parallel.

In den Figuren 3 und 4 sind gleiche Elemente mit gleichen Bezugszeichen versehen.

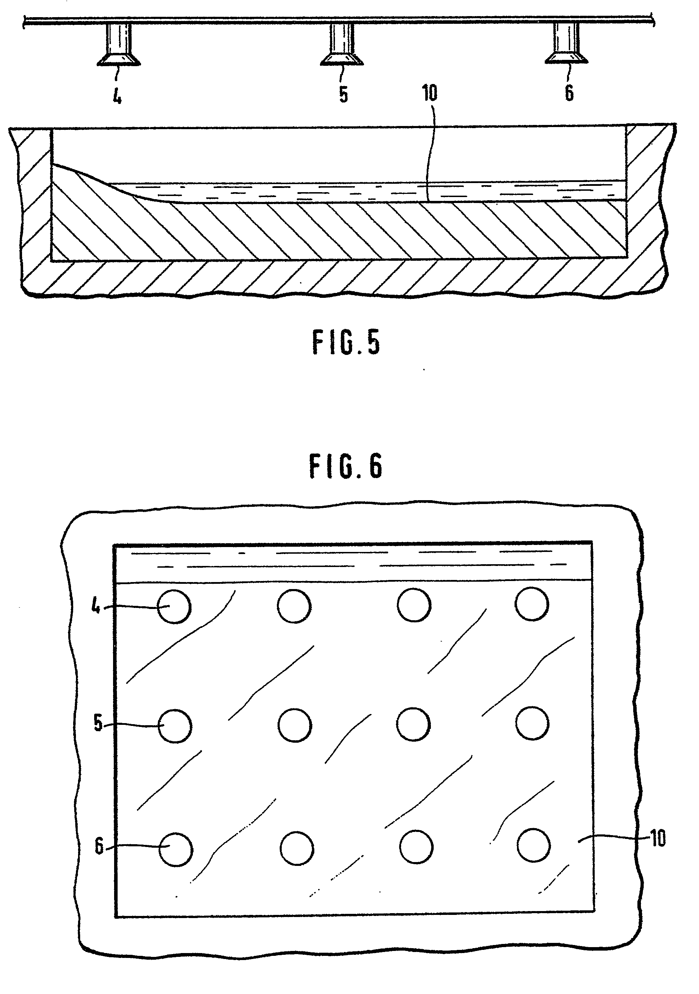

Die Figuren 5 und 6 zeigen ein flaches Becken 10 im Querschnitt (Fig. 5) bzw. in Draufsicht (Figur 6), welches lediglich als Liegefläche ausgebildet ist und eine Wassertiefe von 10 bis 50 cm aufweist. Die Strahler 4, 5 und 6, sowie die sich anschließenden und nicht näher bezeichneten Strahlerreihen leuchten die Liegeflächen des Beckens 10 gleichmäßig aus.

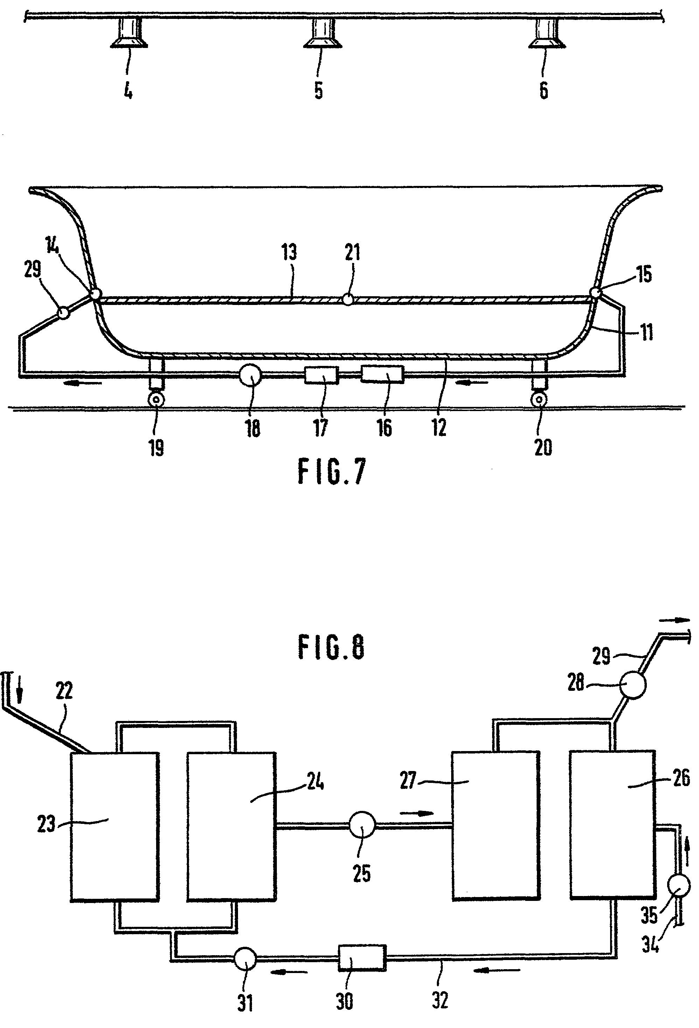

Die Figur 7 zeigt ein weiteres Ausführungsbeispiel der Erfindung in .Form einer fahrbaren Solebadvorrichtung zur Durchführung des Verfahrens nach der Erfindung. Diese besteht aus einer Badewanne 11, hergestellt aus einem säurebeständigen Material, vorzugsweise Kunststoff. Oberhalb des Wannenbodens 12 ist eine Liegefläche 13 angeordnet. Im Kopfbereich der Wanne 11 befindet sich ein Flüssigkeitseinlauf 14 und im Fußbereich der Wanne 11 ein Flüssigkeitsauslauf 15, welche über einen Wärmetauscher 16, eine Filtervorrichtung 17 und eine Pumpe 18 miteinander verbunden sind. Die über der Badewanne 11 angeordneten Strahlenquellen 4, 5 und 6 sind in nicht näher dargestellter Weise höhenverstellbar ausgebildet, so daß sich damit die Strahlungsdichte auf der zu bestrahlenden Fläche in einfacher Weise verändern läßt.

Unmittelbar unter der Liegefläche 13 können in einer nicht näher dargestellten Ausführungsform zusätzlich Strahlenquellen angeordnet sein, wenn die Liegefläche 13 beispielsweise aus Acrylglas hergestellt wird.

Eine solche in Figur 7 dargestellte Anlage wird vorzugsweise im privaten Bereich eingesetzt.

In der Einzelausführung der Wanne, welche für den privaten Gebrauch bestimmt ist, können einzelne Elemente des Systems auch direkt unter der Wanne untergebracht werden. Hierzu gehören bespielsweise Pumpen, Filter, Heizungseinrichtungen und Ventile.

Die Wanne 11 kann auf Rollen 19, 20 gelagert sein und der Flüssigkeitsein- und Flüssigkeitsauslauf 14 bzw. 15 sind in diesem Falle mit nicht näher dargestellten beweglichen Schlauchverbindungen versehen.

Für die gewerbliche Nutzung sind mehrere Wannen in verschiedenen Räumen eingebaut und werden zentral von der Brauchwasseraufbereitungsanlage mit Badewasser versorgt.

Die Figur 8 zeigt die Einrichtung zur Brauchwasser-Aufbereitung. Das aus einem Becken oder einer oder mehreren Wannen aus der Entleerungsleitung 21 abfließende Brauchwasser gelangt über die Zuflußleitung 22 (Fig. 8) in den Brauchwassertank 23, dem ein weiterer Brauchwassertank 24 parallel geschaltet ist. Eine mit einem Filter kombinierte Pumpe 25 pumpt die gefilterte Sole in entsprechende Vorratstanks 26 und 27, welche ebenfalls zueinander parallel geschaltet sind. Mittels einer Pumpe 28 wird die Sole bei Bedarf über die Leitung 29 abgerufen und beispielsweise über den Flüssigkeigkeitseinlauf 14 einer Badewanne 11 (Fig. 7) in der erforderlichen Temperatur zugeführt. Ist die Wanne 11 gefüllt, schaltet ein Magnetventil ab. Von dem Vorratstank 27 der Brauchwasseraufbereitungsanlage erfolgt ein ständiger Rückfluß über die Leitung 32, die thermostatisch gesteuerte Heizung und mit Hilfe der Pumpe 31 in die Brauchwassertanks 23 und 24, so daß eine ständige Aufbereitung der Sole erfolgt. Der Vorrats- oder Aufbereitungstank 26 bzw. 27 ist mit einer Frischwasserleitung 34 verbunden, die über ein Magnetventil 35 stets einen konstanten Wasserstand garantiert.

Der Solegehalt wird durch besondere Meßeinrichtungen im Aufbereitungstank ständig überprüft, um im Bedarfsfall Badesalze in den Aufbereitungstank nachfüllen zu können.

Die Speichertanks 26 und 27 verfügen ständig über eine den gesundheitlichen Richtlinien entsprechend aufbereitete Sole in einer ausreichenden Menge, um entsprechend viele Becken oder Wannen versorgen zu können.

Nach Füllung einer Wanne 11 wird deren eigene Umwälzanlage über einen Schalter eingeschaltet und die Pumpe 18 pumpt das Solewasser durch die Heizungsanlage 16 und hält somit das Wasser für den Benutzer auf eine konstante Temperatur. Nach der Benutzung wird die Wanne über die Leitung 21 entleert, in dem das Wasser abgepumpt wird oder im freien Fall in den Tank 23 fließt (Fig. 8), um erneut aufbereitet zu werden.

Mit der Erfindung werden entsprechende Vorrichtungen angegeben, welche nicht nur in Kliniken zur erfolgreichen Behandlung von Hautkrankheiten nützlich sind, wie Psoriasis, Akne, Ichthyosis (Fischschuppenkrankheit), Kopfschuppenflechte, Neurodermitis, sondern auch in privater Heimbehandlung.

Die Einrichtungen sind aber auch zur Schönheitspflege geeignet, da bei der Bestrahlung mit beispielweise den bekannten Sonnenbänken zur Bräunung die Haut leicht austrocknet, während durch die in zeitlichen Abständen durchgeführten Benetzungen mit der Sole eine vorteilhafte Aktivierung der Haut erfolgt, so daß nicht nur ein Austrocknen der Haut verhindert wird, sondern eine zusätzliche Versorgung der Haut und des Körpers durch die Haut mit wichtigen Salzen erfolgt.

**Patentansprüche**

1. Vorrichtung zur balneo-phototherapeutischen Behandlung von Haut- oder rheumatischen Erkrankungen und/oder kosmetischen oder prophylaktischen Hautbehandlung, dadurch gekennzeichnet, daß die Vorrichtung mindestens einen Strahler (4, 5, 6) mit einem Spektrum, welches den Spektralbereich von 290–420 nm umfaßt und mit einer Solebadvorrichtung (1, 2, 3, 11), deren Sole Spurenelemente sowie einen Salzgehalt von 5–20% und eine Temperatur von 20–38°C aufweist, und die eine Wanne (11) aus einem säurebeständigen Material umfaßt, in der oberhalb des Wannenbodens eine fest eingebaute oder herausnehmbare Liegefläche (2, 13) angeordnet ist, so daß ein Becken mit einer Wassertiefe von 10–50 cm gebildet wird, wobei der oder die Strahler (4, 5, 6) derart angeordnet sind, daß die Liegefläche (2, 13) der Solebadvorrichtung (1, 2, 3, 11) gleichmäßig ausgeleuchtet ist.

2. Vorrichtung nach Anspruch 1, dadurch gekennzeichnet, daß die Zusammensetzung des Solebades bei einer Salinität von 299,89 die folgende Ionenkonzentration in mg pro Liter Wasser, mit einem Spielraum von +/–20% aufweist:

| Magnesium | 36,15 |
|---|---|
| Calcium | 13,78 |
| Natrium | 38,51 |
| Kalium | 7,26 |
| Strontium | 0,24 |
| Lithium | 0,01 |
| Chlorid | 194,44 |
| Bromid | 4,56 |
| Sulfat | 0,72 |
| Hydrogencarbonat | 0,23 |
| Sonstige Salze | 1,12 |

3. Vorrichtung nach Anspruch 1, dadurch gekennzeichnet, daß das Solebad bei einem spezifischen Gewicht von 1,2 bis 1,3 die folgende Zusammensetzung in g pro Liter Wasser, mit einem Spielraum von +/–20% aufweist:

| Magnesiumchlorid | 280 |
|---|---|
| Calciumchlorid | 80 |
| Natriumchlorid | 25 |
| Brom | 8,3 |
| Calciumsulphat | 0,1 |
| Kaliumchlorid | 23 |

4. Vorrichtung nach Anspruch 1, dadurch gekennzeichnet, daß sich im Kopfbereich der Wanne (11) ein Flüssigkeitseinlauf (14) und im Fußbereich der Wanne (11) ein Flüssigkeitsauslauf (15) zur Umwälzung der Sole befinden.

5. Vorrichtung nach Anspruch 1–4, dadurch gekennzeichnet, daß bei festem Einbau der Liegefläche zwischen Wannenboden und Liegefläche eine Pumpe mit angeschlossener thermostatisch geregelter Heizung angeordnet ist.

6. Vorrichtung nach Anspruch 1 oder einem der voranstehenden, dadurch gekennzeichnet, daß der Flüssigkeitsauslauf (15) der Solebadvorrichtung (11) über eine Filtervorrichtung (17), einen Aufbereitungstank (23, 24), einen thermisch isolierten Speichertank (26, 27), eine thermostatisch gesteuerte Heizung (30) und eine Pumpvorrichtung (31) mit dem Flüssigkeitseinlauf (14) der Solebadvorrichtung (11) verbunden ist.

7. Vorrichtung nach Anspruch 6, dadurch gekennzeichnet, daß der Speichertank (26, 27) mit einem Frischwasserzulauf (34) und einem Abwasserablauf (32) über steuerbare Ventile (35) verbunden ist.

8. Vorrichtung nach Anspruch 1 oder einem der voranstehenden, dadurch gekennzeichnet, daß mehrere Solebadvorrichtungen (1, 2, 3, 11), insbesondere Wannen (11) zur Aufbereitungs- und Speichervorrichtung (11) über zwischengeschaltete Ventile zueinander parallel geschaltet sind.

9. Vorrichtung nach Anspruch 1 oder einem der voranstehenden, dadurch gekennzeichnet, daß die über der Solebadvorrichtung (1, 2, 3, 11) angeordneten Strahler (4, 5, 6) höhenverstellbar ausgebildet sind.

10. Vorrichtung nach Anspruch 1 oder einem der voranstehenden, dadurch gekennzeichnet, daß die Wanne (11) aus Kunststoff besteht.

11. Vorrichtung nach Anspruch 1 oder einem der voranstehenden Ansprüche, dadurch gekennzeichnet, daß unter der aus Acrylglas bestehenden Liegefläche (13) der Wanne (11) mindestens eine Strahlenquelle (4, 5, 6) angeordnet ist.

12. Vorrichtung nach Anspruch 1 oder einem der voranstehenden Ansprüche, dadurch gekennzeichnet, daß dem Becken (1) der Solebadvorrichtung ein Bewegungsbecken (3) angeschlossen ist.

**Claims**

1. Device for balneo-phototherapeutic treatment of skin or rheumatic disorders and/or cosmetic or prophylactic skin treatment, characterised in that the device consists of at least one radiator (4, 5, 6) with a spectrum which covers the spectral area of 290–420 nm and with a brine reservoir device (1, 2, 3, 11), the brine of which has trace elements as well as a salt content of 5–20% and a temperature of 20–38°C, and which has a bath (11) made of an acid resistant material wherein above the base of the bath a firmly fixed or removable lying surface (2, 13) is arranged so that a pool with a water depth of 10–50 cm is formed whereby the radiator(s) (4, 5, 6) is (are) arranged in such a way that the lying surface (2, 13) of the brine reservoir device (1, 2, 3, 11) is illuminated evenly.

2. Device according to claim 1, characterised in that the composition of the brine reservoir has in the case of a salinity of 299,89 the following ion concentration in mg per litre of water, with a margin of +/–20%:

| | |
|---|---|
| Magnesium | 36.15 |
| Calcium | 13.78 |
| Sodium | 38.51 |
| Potassium | 7.26 |
| Strontium | 0.24 |
| Lithium | 0.01 |
| Chloride | 194.44 |
| Bromide | 4.56 |
| Sulphate | 0.72 |
| Hydrogen carbonate | 0.23 |
| Other salts | 1.12 |

3. Device according to claim 1, characterised in that the brine reservoir has in the case of a specific gravity of 1.2 to 1.3 the following composition in g per litre of water, with a margin of +/–20%:

| | |
|---|---|
| Magnesium chloride | 280 |
| Calcium chloride | 80 |
| Sodium chloride | 25 |
| Bromide | 8.3 |
| Calcium sulphate | 0.1 |
| Potassium chloride | 23 |

4. Device according to claim 1, characterised in that in the hand area of the bath (11) there is a fluid inlet (14) and in the foot area of the bath a fluid outlet (15) for the circulation of the brine.

5. Device according to claims 1–4, characterised in that if the lying surface is securely fastened a pump with attached thermostatically regulated heating is arranged between the base of the bath and the lying surface.

6. Device according to claim 1 or one of the preceding claims, characterised in that the fluid outlet (15) of the brine reservoir device (11) is connected by means of a filtering device (17), a treatment tank (23, 24), a thermally insulated storage tank (26, 27), a thermostatically controlled heating device (30) and a pump device (31) with the fluid outlet (14) of the brine reservoir device (11).

7. Device according to claim 6, characterised in that the storage tank (26, 27) is connected with a fresh water inlet and a waste water outlet by means of controllable valves.

8. Device according to claim 1 or one of the preceding claims, characterised in that several brine reservoir devices (1, 2, 3, 11) in particular baths (11) to the treatment and storage device (11) are connected in parallel by means of intermediate valves.

9. Device according to claim 1 or one of the preceding claims, characterised in that the radiators (4, 5, 6) arranged above the brine reservoir device (1, 2, 3, 11) are adjustable in height.

10. Device according to claim 1 or one of the preceding claims, characterised in that the bath (11) is made of plastic.

11. Device according to claim 1 or one of the preceding claims, characterised in that beneath the lying surface (13) of the bath which is made of acrylic glass at least one radiation source (4, 5, 6) is arranged.

12. Device according to claim 1 or one of the preceding claims, characterised in that an exercise pool (3) is connected to the pool (1) of the brine reservoir device.

**Revendications**

1. Dispositif pour le traitement balnéo-photothérapeutique d'affections cutanées ou rhumatismales et/ou le traitement cosmétique ou prophylactique de la peau, caractérisé en ce que le dispositif comporte au moins une source de rayonnement (4, 5, 6) avec un spectre qui couvre le domaine spectral de 290–420 nm et avec un dispositif pour bain d'eau salée (1, 2, 3, 11), dont la saumure présente des éléments de trace, ainsi qu'une teneur en sel de 5–20% et une température de 20–38°C, et qui comporte une baignoire (11) constituée d'une matière résistant aux acides, dans laquelle, au-dessus du fond de la baignoire, une surface de repos (2, 13) fixe ou amovible est disposée de telle sorte qu'un bassin d'une profondeur d'eau de 10–50 cm est créé, la (les) source(s) de rayonnement (4, 5, 6) étant disposée(s) de telle sorte que la surface de repos (2, 13) du dispositif pour bain d'eau salée (1, 2, 3, 11) soit illuminée de façon uniforme.

2. Dispositif selon la revendication 1, caractérisé en ce que la composition du bain d'eau salée, pour une salinité de 299,89, présente la concentration en ions suivante en mg par litre d'eau, avec une marge de 20%:

| | |
|---|---|
| Magnésium | 36,15 |
| Calcium | 13,78 |
| Sodium | 38,51 |
| Potassium | 7,26 |
| Strontium | 0,24 |
| Lithium | 0,01 |
| Chlorure | 194,44 |
| Bromure | 4,56 |
| Sulphate | 0,72 |
| Hydrocarbonate | 0,23 |
| Autres sels | 1,12 |

3. Dispositif selon la revendication 1, caractérisé en ce que le bain d'eau salée, pour un poids spécifique de 1,2 à 1,3, présente la composition suviante en g par litre d'eau, avec une marge de 20%:

| | |
|---|---|
| Chlorure de magnésium | 280 |
| Chlorure de calcium | 80 |
| Chlorure de sodium | 25 |
| Brome | 8,3 |
| Sulfate de calcium | 0,1 |
| Chlorure de potassium | 23 |

4. Dispositif selon la revendication 1, caractérisé en ce que, à la tête de la baignoire (11), on trouve une arrivée de liquide (14) et, au pied de la baignoire (11), une évacuation de liquide (15) pour lacirculation de l'eau salée.

5. Dispositif selon les revendications 1–4, caractérisé en ce que, pour un montage fixe de la surface de repos, on prévoit une pompe avec chauffage à réglage thermostatique, entre le fond de la baignoire et la surface de repos.

6. Dispositif selon la revendication 1 ou l'une des revendications qui précèdent, caractérisé en ce que l'évacuation de liquide (15) du dispositif à bain d'eau salée (11) est reliée via un dispositif de filtration (17), un réservoir de préparation (23, 24), un réservoir de stockage à isolation thermique (26, 27), un chauffage à commande par thermostat (30) et un dispositif de pompage (31) avec l'arrivée de liquide (14) du dispositif à bain d'eau salée (11).

7. Dispositif selon la revendication 6, caractérisé en ce que le réservoir de stockage (26, 27) est relié à une arrivée d'eau fraîche (34) et à une évacutation d'eau (32) par des vannes commandées (35).

8. Dispositif selon la revendication 1 ou l'une des revendications qui précèdent, caractérisé en ce que plusieurs dispositifs à bain d'eau salée (1, 2, 3, 11), en particulier des baignoires sont reliées en parallèle, avec intercalation de vannes, au dispositif de préparation et de stockage (11).

9. Dispositif selon la revendication 1 ou l'une des revendications qui précèdent, caractérisé en ce que les sources de rayonnement (4, 5, 6) disposées au-dessus du dispositif à bain d'eau salée (1, 2, 3, 11) sont configurées de manière à être réglables en hauteur.

10. Dispositif selon la revendication 1 ou l'une des revendications qui précèdent, caractérisé en ce que la baignoire (11) est en matière synthétique.

11. Dispositif selon la revendication 1 ou l'une des revendications qui précèdent, caractérisé en ce qu'une source de rayonnement (4, 5, 6), au moins, est disposée sous la surface de repos, (13) consistant en verre acrylique, de la baignoire (11).

12. Dispositif selon la revendication 1 ou l'une des revendications qui précèdent, caractérisé en ce qu'un bassin de circulation (3) est raccordé au bassin (1) du dispositif à bain d'eau salée.

# FIG.1

# FIG.2

# FIG.3

# FIG.4

FIG.5

FIG.6

**F I G. 7**

**F I G. 8**